# EUROPEAN PATENT SPECIFICATION

(11) **EP 0 625 897 B1**
(45) Date of publication and mention of the grant of the patent: **11.09.1996**
(21) Application number: 93903293.4
(22) Date of filing: 22.01.1993
(51) Int. Cl.: A61H 3/00, A61B 5/103

(54) **APPARATUS FOR THE ANTIGRAVITY MODIFICATION OF THE MYOTENSIONS ADAPTING THE HUMAN POSTURE IN ALL OF THE THREE PLANES OF THE SPACE**
DIE KÖRPERHALTUNG IN ALLEN DREI RAUMEBENEN ANPASSENDE VORRICHTUNG ZUR MODIFIKATION DER MYOTONIEN
APPAREIL DE MODIFICATION ANTIGRAVIFIQUE ET TRIDIMENSIONNELLE DES TENSIONS MUSCULAIRES ASSURANT L'ADAPTATION POSTURALE DU CORPS HUMAIN

(30) Priority: 23.01.1992 IT RM920051
(43) Date of publication of application: 30.11.1994
(73) Proprietor: GIOVANNETTI, Giovanni, Battista, I-00139 Roma (IT)
(72) Inventor: GIOVANNETTI, Giovanni, Battista, I-00139 Roma (IT)
(74) Representative: Sarpi, Maurizio
(86) International application number: IT9300005
(87) International publication number: WO9314733

(56) References cited:
- EP-A- 0 002 188
- DE-A- 2 151 933
- DE-U- 9 017 709
- FR-A- 2 252 108
- BIOMEDIZINISCHE TECHNIK vol. 32, no. 10, October 1987, BERLIN (D) pages 250 - 254 G. A. HORTSMAN ET AL. 'Speziallaufband f r Stand- und Ganguntersuchungen in Forschung und Klinik' the whole article

## Description

The present invention relates to an apparatus for re-educating the posture of the backbone of an individual in all of the three plane of the space, i.e. frontal, sagittal, and transversal, thus avoiding the posture defects and all of the pathologic consequences arising threfrom, under antigravity, dynamic conditions and through deambulation on a travelling band.

As known, the weight of the human body is supported by the skeleton, muscles, ligaments, and fasciae. The line representing that weight goes down from above along the backbone, bifurcates at the third lumbar vertebra, turns to the two opposite coxae (hip-joints), goes down along the lower limbs and goes up again by the same way until it rejoins at the centre of the pubic bone to form a closed loop of forces. However, skeleton is very seldom postured in a physiologically correct way so as to absorb the most of the weight of the human body. Actually men assume comfort postures or postural bad habits requiring the aid of supports constituted by muscular tensions, fasciae, and ligaments to restore the balance. Such "dynamic compensations" of a "static defect" become permanent in time such as to constitute a pseudo scheme of movement (the automatic total scheme of movements) that, even if it is not physiologically correct, becomes definitively functional, thus causing pathologic modifications in capsules, ligaments and joint surfaces and occurrence of aches and pains of different nature and intensity.
The physiatrics is aware of such problem for some time so that several equipment have been manufactured to try to solve it. The latter, however, have never considered the "global problem", i.e. the functional and dynamic whole of the human body.

From FR-A-2 252 108 it is known an apparatus for re-education of the human backbone comprising means for carrying out differential traction at different levels, means for correcting the postural defects and for blocking the patient and means for allowing the patient to walk. However this apparatus does not permit both a diagnostics investigations of postural defects and a programmed treatment since there is no means of monitoring the orthostatic position of the patient. It is already known from the Biomedizinische Technik Vol.32 October 1987, the possibility to control the weight resting on each patient's foot lying on a footboards to investigate the standing and walking motor control and to value neuromuscular diseases without the possibility of corrections.

The present invention seeks to overcome the limits of the existing apparatus and to provide an apparatus by which the correction of postural bad habits upstream of the several peripheral symptoms which can occur in different ways becomes total, permanant, spontaneous and directed in all of the three planes of the space.
The inventive concept of the present invention is of providing an apparatus by which the patient can be subjected to differential tractions at different levels by cancelling out all or a part of his weight and effecting a number of successive corrections such as to force the patient to assume a correct posture in the frontal, sagittal, and transversal planes in static attitude, and by forcing the patient to keep such posture even in dynamic attitude, i. e. during the deambulation, until he reaches stably and definitevely a new correct total scheme of movements, while keeping the orthostatic position of the patient under continuous monitoring. According to the invention this can be achieved by continuously monitoring the weight resting on either patient's foot whose unbalance shows postural defects of the subject or additionally by detecting the displacements of reference points of the patient's body with respect to an ideal line.
Therefore, the present invention provides according to a first aspect thereof a diagnostic and therapeutic apparatus for re-educating the posture of the backbone which includes in combination means for monitoring the orthostatic posture of the patient by measuring the weight resting on each patient's foot, means for carrying out a differential traction on the same patient at different levels in order to cancell out all or a part of his weight, means for correcting the postural defects by leading the patient again to the respect of the symmetries in the three planes of the space and for blocking the patient within postural limits inside which he is free but from which he cannot come out, means for allowing the patient to walk in his tridimensionally corrected hanging posture in order to force him to the new automatic scheme of movements adapted to the incidental situation, wherein the means for controlling the weight resting on each foot feeds data to a microprocessor controlling automatically the operation of the traction means for leading the patient again to a predetermined postural scheme.
A further object of the invention is of providing an apparatus as described above, wherein the data of the displays is transmitted to a computer adapted to store the data of the beginning and the end of the treatment and to issue a graphic representation of the same.
Still another object of the invention is of providing an apparatus including, optical and/or electronic means for detecting the spatial variation of the position of a reference point attached to the patient's body.

According to a first preferred embodiment the apparatus of the present invention includes in combination a travelling band for the deambulation provided with two footboards sensitive to changes in pressure and adapted to indicate on a display the weight resting on each foot of the patient standing thereon, and a rigid framework which can be firmly anchored to the floor and/or to the ceiling of the operational room above said travelling band, and to which the patient is secured by a number of traction slings fastened to five suspension points, e.g. pulleys. Such slings allow both the displayed data to be set, i.e. the weight resting on both feet to be compensated, and such weight to be partially released in a symmetric way at different levels so as to display the wrong postural attitudes of the patient through the loss of the previously reached balance between the weights resting on the two feet.
The apparatus also includes a number of movable arms provided with retainers or stops to be applied to the patient's body at different (pelvic, sacral, sternal, humeral and dorsal) levels in order to prevent him from assuming postures different from those therapeutic even during the deambulation. According to a preferred embodiment such retainers consist of oleodynamic pistons assembled in suitable hydraulically driven structures and controlled for an optimum positioning.

Further embodiment of the invention are disclosed in the dependent claims.

The description of the invention will be better understood with reference to the accompanying drawings which illustrate only by way of a non-limitative example a preferred embodiment thereof.
In the darwings:
Fig. 1 is a perspective view of the apparatus of the invention;
Fig. 2 is a schematic elevation side view of the same;
Fig. 3 is a plan view of a detail of one arm provided with retainers;
Figs. 4 and 5 are a frontal elevation view and a side view of a second embodiment of the invention, respectively, in which the retainers are oleodinamically driven;
Fig. 6 is a detail of a movable framework where oleodynamic cylinders are provided.

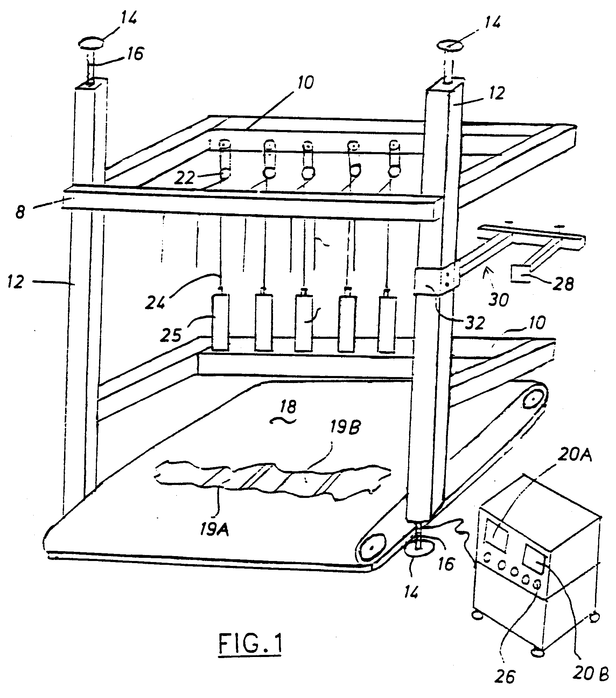

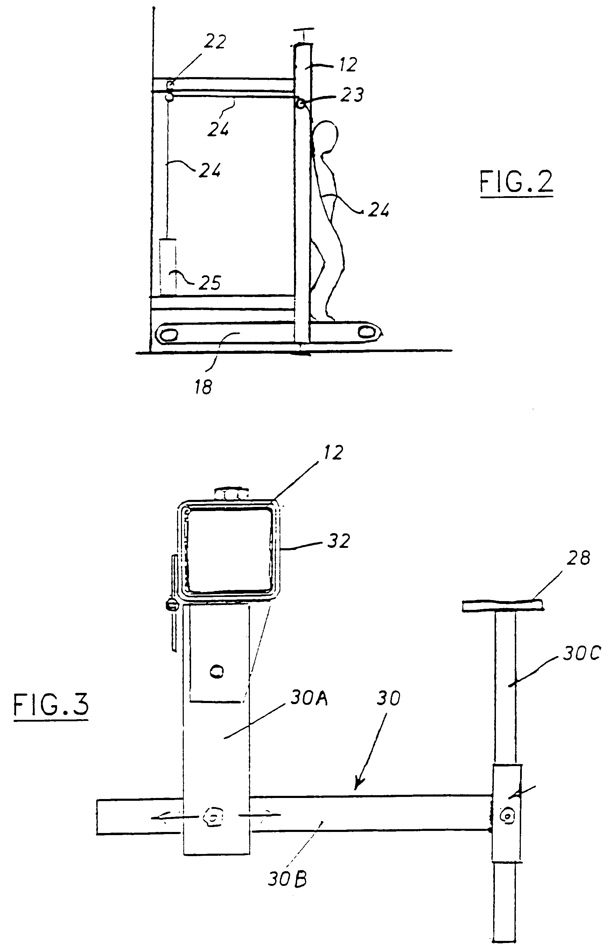

With reference to Figs. 1 to 3 the apparatus includes a bridge framework of tubular metal sections formed by a front beam 8, a rear beam or a pair of rear beams 10, and two uprights 12 extending from the floor to the ceiling of the operational room and secured thereto by mutual abutment of pressure discs 14 adjustable through threaded pins 16 without drilling or permanent securing devices. A travelling band 18 for deambulation having a varying resistance is placed between the two uprights 12 on two footboards or load cells 19A and 19B sensitive to changes in pressure and each adapted to transmit to a corresponding display 20A and 20B the weight resting on each patient's foot.
Attached to the rear beam 10 are a certain number (five in the illustrated example) of pulleys 22 around which traction lacings 24 are wound which are subjected to tension by devices 25 of the known type such as electric or hydraulic pistons, counterweight systems of conventional mechanical (rocker arm) or hydraulic type, or other equivalent systems shown only schematically. The traction values are displayed by suitable indicators to the operator. Such lacings pass around pulleys 23 of front beam 8 and are provided with the ordinary accessories used in the traction techniques such as traction bands and slings, length regulators, a.s.o. The apparatus is also provided with a number of rigid retainers or stops 28 to be attached to the patient's body for its blocking in the posture forced by the correction traction means. Such retainers are carried by horizontal arms 30 which can vertically slide along uprights 12 by means of sleeves 32 and be adjusted in their lengths as they are formed of several pieces 30A. 30B and 30C slidable perpendicularly to one another. The described apparatus has both diagnostic and therapeutic functions. It is thereby possible to show first of all the comfort postures or postural defects of the subject and then to lead the same subject to take on correct postures which becomes stable and definitive as the therapy proceeds.
It is now disclosed by way of example the treatment of a subject having five suspension points, and namely: one at the cranium, two at the thorax, and two at the ischium (inguen).
The patient is placed on travelling band 18 with his feet resting exactly on the corresponding footboards sensing the weight. The right display 20A shows the weight resting as a whole on the right foot and the left display 20B shows the weight resting as a whole on the left foot. Such weights are almost different from each other because the total weight divides equitably very seldom but rests more on the right side or the left side than the other side according to the importance of the posture defect to be corrected.
The right or left ischiatic suspension acts for levelling the values of the two displays 20 through the corresponding traction lacing 24. This operation aims at balancing the pelvis to avoid resting defects on one side or on the other side.
Now a symmetric thoracic traction, i.e. a balanced weight release on the right and left sides, is carried out in order to compensate about 20% of the body weight. The preceding values of displays 20 are very likely varied at the end of such operation. Actually, if the subject had, for example, a posture of the trunk inclined to the right side due to the rotation of the backbone or to the retraction of the muscles responsible for that movement towards that side, the indifferentiated upwards traction of the trunk will raise the pelvis on that side and will deduct a weight equal to the intensity and amplitude of the postural lack of balance.
The traction is then differentiated by means of a lateral thoracic tie-rod 24 so as to compensate the values of displays 20.
Finally, a light cervical traction is carried out for excluding the weight of the cranium: the patient is then subjected to differential tractions at several levels, the intensity thereof expressing the existing myofascial reactions.
The pelvic, sacral, sternal, humeral and dorsal retainers or stops 28 are then fastened to the patient's body in order to discourage any attempt of compensation by an attitude other than that forced by the correcting tractions.
Patient is invited to walk by setting a very low resistance at the beginning.
It is self-evident that under such conditions the patient cannot make use of his usual (and wrong) scheme of deambulation as:
- his proprioceptive sensations of his own weight are deeply changed and then he cannot make use of his usual scheme of movement;
- the attempts of compensation made by the assumption of different, less tiring, comfort postures are cancelled by the differential tractions of lacings 24 and by stops 28 fixing the therapeutic posture.
Any pressure sensor on stops 28 can display the different thrusts of the several points of the body and then the attitude against a back thrust which rotates the backbone.
Under such conditions the subject is forced to invent a new way of movement and shall do this by using skeleton, muscles, a.s.o. which are now correctly postured. If at the beginning he moves with extreme difficulty, as the therapy proceeds the subject will stably reach a new global, now correct scheme of movement.
The displays can translate into objective data the progress of the therapy. In fact, as the therapy proceeds and the several defective bends of the backbone and the myofascial reactions are compensated and cancelled, it is as though the trunk of the patient got longer. As a consequence the efficiency of the thoracic traction will be progressively reduced, and the displays will show it by an increase of the weight shown. The responsible personnel can then draw up a hospital file of the patient in which the detected values in the several therapy sessions will be recorded until stable values not subjected to change are reached. Thus a graph as well as digital data of the reached, global and definitive, correct posture can be obtained.
The described apparatus is susceptible to be used in combination with a microprocessor capable of storing treatment beginning and end data, the evaluation of the differences detected, and data during the treatment to statistic research purposes. It is also possible to use a microprocessor for controlling the function on the base of a stored program.
Alternatively, the orthostatic posture of the subject can be put under control by detecting the displacement of a reference point of the patient's body in the space. To this purpose a photoelectric cell and a scanner can be used.

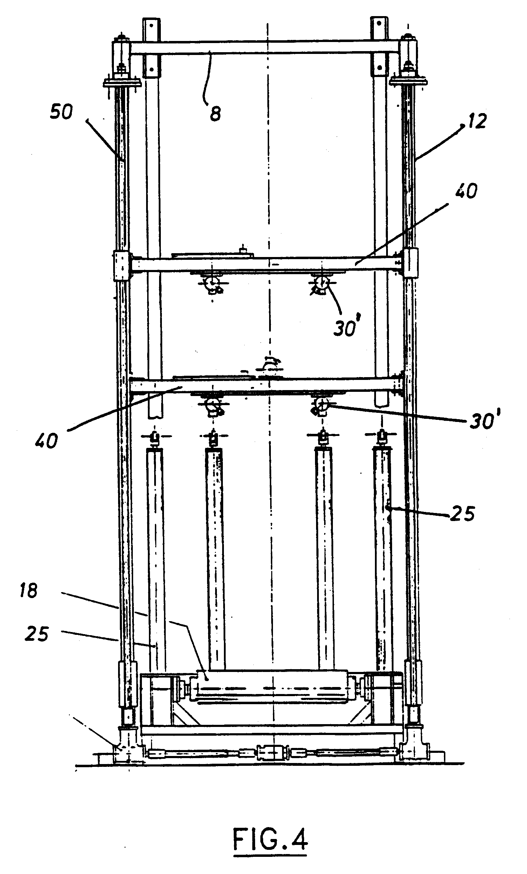

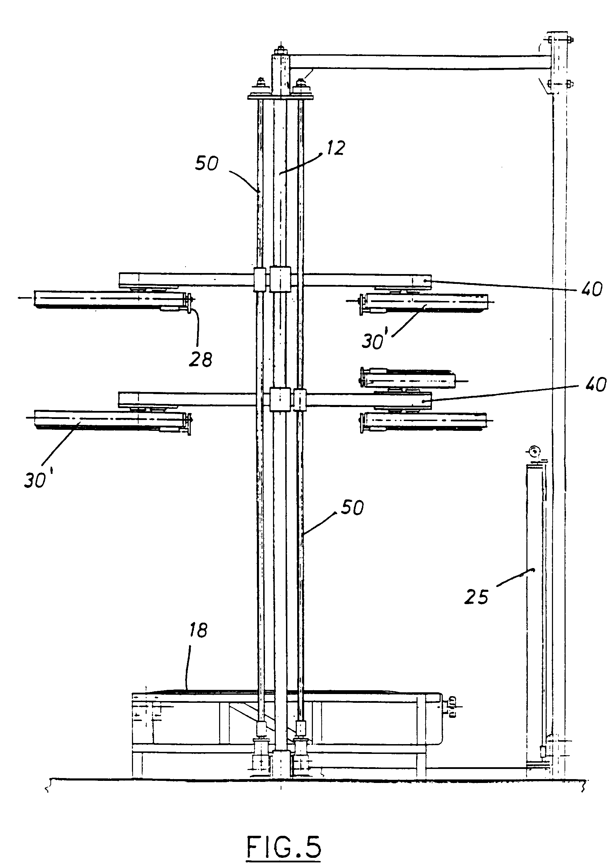

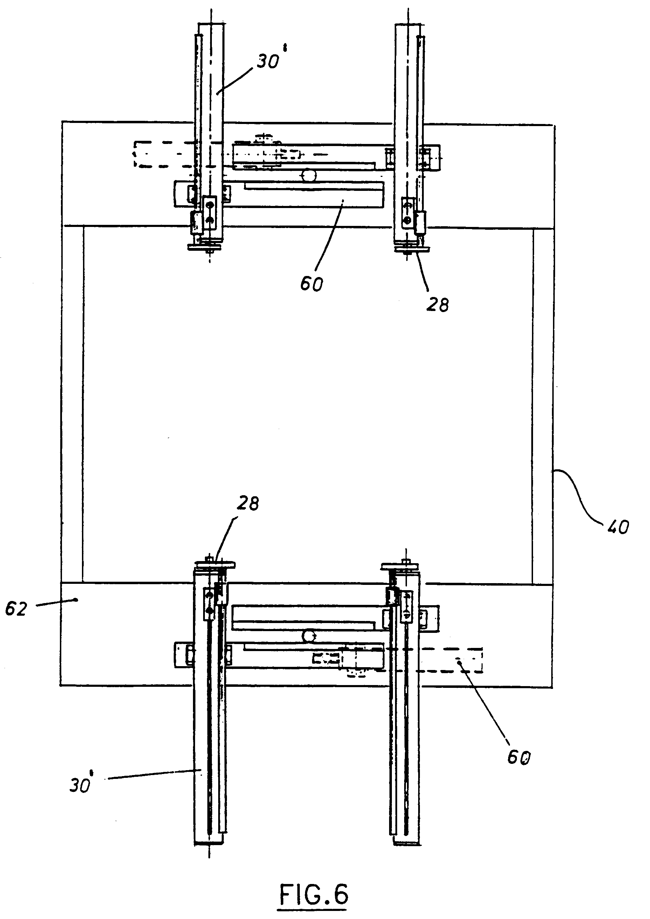

In Figs. 4 to 6 a second embodiment is shown, in which stops 28 are moved by oleodynamic cylinders 30' carried on frames 40 vertically movable along guides 50 parallel to uprights 12' between which slidable footboard 18 is placed. As can be seen, cylinders 30' can be horizontally displaced on the relative movable frames 40 and approached to or moved away from each other by a further oleodynamic positioning cylinder 60 attached to a proper plate 62.

## Claims

1. A diagnostic and therapeutic apparatus for reeducating the posture of the human backbone comprising, in combination, means (24, 25) for carrying out a differential traction on the same patient at different levels in order to cancell out all or a part of his weight, means (28, 30, 32, 12) for correcting the postural defects by leading the patient again to the respect of the symmetries in the three planes of the space and for blocking the patient within postural limits inside which he is free but from which he cannot come out, and means (18) for allowing the patient to walk in his tridimensionally corrected hanging posture in order to force him to the new automatic scheme of movements adapted to the incidental situation, characterized in that it further comprises means (19A, 19B) for monitoring the orthostatic posture of the patient by measuring the weight resting on each foot of a standing or walking subject, and a microprocessor and in that the means (19A, 19B) for measuring the weight resting on each foot of the subject is adapted to feed data to the microprocessor controlling automatically the operation of the traction means (24, 25) for leading the subject to a predetermined postural scheme.

2. The diagnostic and therapeutic apparatus as claimed in the preceding claims, characterized in that the means (19A, 19B) for measuring the weight resting on each foot of the subject are footboards sensitive to changes in pressure or load cells placed under the walking surface of a travelling or slidable band (18).

3. The diagnostic and therapeutic apparatus as claimed in the preceding claims, characterized in that the traction means are lacings or traction slings (24) adjustable in length and slidable around pulleys (22) carried by a frame (8, 10, 12) which can be firmly anchored to the floor and/or to the ceiling of the operational room, and put under tension by electric, mechanical or hydraulic devices (25).

4. The diagnostic and therapeutic apparatus as claimed in the preceding claims, characterized in that said traction slings (24) are designed to the treatment of the subject through five suspension points, and namely one at the cranium, two at the thorax, and two at the ischium (inguen).

5. The diagnostic and therapeutic apparatus as claimed in the preceding claims, characterized in that the means (28, 30, 32, 12) for correcting the postural defects and for blocking the subject within postural limits are a number of stops (28) which are placed against the patient's body at the predetermined points by vertically shifting and adjusting rigid support arms (30) slidable by means of sleeves (32) along the uprights (12) of the apparatus.

6. The diagnostic and therapeutic apparatus as claimed in the preceding claims, characterized in that said stops (28) are provided with pressure sensors for measuring the thrust of the subject.

7. The diagnostic and therapeutic apparatus as claimed in the preceding claims, characterized in that said stops (28) are carried by oleodynamic pistons (30') attached to rigid, hydraulically driven support structures (40) which can be properly moved and positioned.

8. The diagnostic and therapeutic apparatus as claimed in the preceding claims, characterized in that an electronic central processing unit is provided which is adapted to receive data detected by the load cells (19A, 19B) of the travelling band (18) and the values of the tractions at the several levels of the body during the therapy so as to plot the whole treatment.

9. The diagnostic and therapeutic apparatus as claimed in the preceding claims, characterized in that a device detecting the position of a reference point attached to the patient's body in the space is additionally used for monitoring the orthostatic posture of the patient.

## Patentansprüche

1. Vorrichtung zur Diagnose und zur Therapierung der Wiedererlangung der Haltung der menschlichen Wirbelsäule, mit
Mitteln (24, 25) zur Ausübung einer unterschiedlichen Zugbelastung am gleichen Patienten, jedoch auf unterschiedlichen Höhen, zur teilweisen oder vollständigen Neutralisation dessen Gewichtes,
Mitteln (28, 30, 32, 12) zur Korrektur der Haltungsschäden, indem der Patient mit bezug zu den drei Raumebenen wieder in das Gleichgewicht gebracht wird, sowie zur Festlegung des Patienten innerhalb von Haltungsbereichen, innerhalb deren er sich frei bewegt, die er jedoch nicht verlassen kann, und
Mitteln (18), die es dem Patienten ermöglichen, in seiner dreidimensional korrigierten hängenden Haltung zu gehen, um den Patienten in das neue, sich automatisch ergebende Bewegungsschema zu zwingen, welches der individuellen Situation angepaßt ist, **dadurch gekennzeichnet, daß** die Vorrichtung darüber hinaus
Mittel (19A, 19B) umfaßt, zur Überwachung der orthostatischen Haltung des Patienten, durch Messung des Gewichts, welches auf jedem Fuß eines stehenden oder gehenden Patienten lastet, und
einen Mikroprozessor umfaßt, wobei die Mittel (19A, 19B) zur Messung des Gewichtes, welches auf jedem Fuß des Patienten lastet, so ausgelegt sind, daß sie Daten an den Mikroprozessor weiterleiten, der automatisch den Betrieb der Zugbelastungsmittel (24, 25) regelt, zur Überführung des Patienten in ein vorgegebenes Haltungsschema.

2. Vorrichtung zur Diagnose und zur Therapierung nach Anspruch 1, dadurch gekennzeichnet, daß die Mittel (19A, 19B) zur Messung des Gewichts, welches auf jedem Fuß des Patienten lastet, Trittbretter sind, die bezüglich Änderungen des Druckes empfindlich sind oder Meßdosen sind, die unterhalb der Lauffläche eines Laufbandes oder Gleitbandes (18) angeordnet sind.

3. Vorrichtung zur Diagnose und zur Therapierung nach einem der vorhergehenden Ansprüche, dadurch gekennzeichnet, daß die Zugbelastungsmittel Seile oder Zugseilschlingen (24) sind, die in der Länge einstellbar sind und die um Rollen (22) verlagerbar sind, die von einem Rahmen (8, 10, 12) getragen sind, der fest an dem Boden verankerbar ist und/oder an der Decke des Arbeitsraumes, wobei die Seile mittels elektrischer, mechanischer oder hydraulischer Einrichtungen unter Spannung gehalten sind.

4. Vorrichtung zur Diagnose und zur Therapierung nach einem der vorhergehenden Ansprüche, dadurch gekennzeichnet, daß die Zugseilschlingen (24) zur Behandlung des Patienten an fünf Aufhängungspunkten vorgesehen sind, nämlich einer im Kopfbereich, zwei im Brustbereich und zwei im Lendenbereich.

5. Vorrichtung zur Diagnose und zur Therapierung nach einem der vorhergehenden Ansprüche, dadurch gekennzeichnet, daß die Mittel (28, 30, 32, 12) zur Korrektur der Haltungsschäden sowie zur Festlegung des Patienten innerhalb von Haltungsbereichen durch eine Anzahl von Anschlägen (28) ausgebildet sind, die am Körper des Patienten an vorbestimmten Stellen in Anlage bringbar sind, indem steife Tragarme (30) vertikal verschiebbar und einstellbar vorgesehen sind, wobei diese Verschiebbarkeit mittels entlang der Standfüße (12) der Vorrichtung verschiebbarer Hülsen (32) gegeben ist.

6. Vorrichtung zur Diagnose und zur Therapierung nach einem der vorhergehenden Ansprüche, dadurch gekennzeichnet, daß die Anschläge (28) zur Messung des Andruckes durch den Patienten mit Druckfühlern versehen sind.

7. Vorrichtung zur Diagnose und zur Therapierung nach einem der vorhergehenden Ansprüche, dadurch gekennzeichnet, daß die Anschläge (28) von öldynamischen Kolben (30') getragen sind, die an steifen, hydraulisch angetriebenen Traganordnungen (40) angebracht sind, die geeignet bewegbar und positionierbar sind.

8. Vorrichtung zur Diagnose und zur Therapierung nach einem der vorhergehenden Ansprüche, dadurch gekennzeichnet, daß eine zentrale elektronische Verarbeitungseinheit vorgesehen ist, die die Daten empfängt, die von den Meßdosen (19A, 19B) des Laufbandes (18) erfaßt werden, sowie die Werte der Zugspannungen in den mehreren Höhen am Körper während der Therapie empfängt, so daß die gesamte Behandlung aufzeichenbar ist.

9. Vorrichtung zur Diagnose und zur Therapierung nach einem der vorhergehenden Ansprüche, dadurch gekennzeichnet, daß zusätzlich eine Erfassungseinrichtung zur Erfassung der Lage eines Referenzpunktes im Raum eingesetzt wird, der am Körper des Patienten angebracht ist, zur Überwachung der orthostatischen Haltung des Patienten.

## Revendications

1. Dispositif de diagnostic et de thérapie destiné à la rééducation de la posture de la colonne vertébrale humaine comprenant, de manière combinée, des moyens (24, 25) pour effectuer une traction différentielle sur le même patient à différents niveaux afin de neutraliser tout ou partie de son poids, des moyens (28, 30, 32, 12) destinés à corriger les défauts de posture en conduisant à nouveau le patient au respect des symétries dans les trois plans de l'espace et au blocage du patient à l'intérieur des limites de posture dans lesquelles il est libre mais dont il ne peut pas sortir, et des moyens (18) pour permettre au patient de marcher dans sa posture suspendue corrigée tridimensionnellement afin de l'obliger à suivre le nouveau schéma automatique de mouvements appropriés à la situation correspondance, caractérisé en ce qu'il comprend en outre des moyens (19A, 19B) destinés à surveiller la posture orthostatique du patient en mesurant le poids reposant sur chaque pied du sujet debout ou en train de marcher et un microprocesseur et en ce que le moyen (19A, 19B) destiné à mesurer le poids reposant sur chaque pied du sujet est conçu peur alimenter en données le microprocesseur commandant automatiquement le fonctionnement des moyens de traction (24, 25) pour amener le patient à un schéma prédéterminé de posture.

2. Dispositif de diagnostic et de thérapie selon la revendication précédente, caractérisé en ce que les moyens (19A, 19B) destinés à mesurer le poids reposant sur chaque pied du sujet sont des plateaux de pieds sensibles aux modifications de pression ou des cellules de charge placées sous la surface de marche d'une bande qui se déplace ou qui coulisse (18).

3. Dispositif de diagnostic et de thérapie selon les revendications précédentes, caractérisé en ce que les moyens de traction sont des laçages ou des cordes de traction (24) réglables en longueur et pouvant coulisser autour des poulies (22) supportées par un cadre (8, 10, 12) qui peut être ancré fermement dans le plancher et/ou dans le plafond de la pièce opérationnelle, et mis sous tension au moyen de dispositifs électrique, mécanique ou hydraulique (25).

4. Dispositif de diagnostic et de thérapie selon les revendications précédentes, caractérisé en ce que lesdites cordes de traction (24) sont conçues pour le traitement du sujet avec cinq points de suspension, et à savoir l'un étant au niveau du crâne, deux au niveau du thorax et deux au niveau de l'ischion (inguen).

5. Dispositif de diagnostic et de thérapie selon les revendications précédentes, caractérisé en ce que les moyens (28, 30, 32, 12) destinés à corriger les défauts de posture et à bloquer le sujet à l'intérieur de limites de posture sont composés d'un certain nombre de butées d'arrêt (28) qui sont placées contre le corps du patient au niveau des points prédéterminés déplaçant verticalement et en réglant les arbres rigides des supports (30) qui peuvent coulisser au moyen des manchons (32) le long des colonnes (12) du dispositif.

6. Dispositif de diagnostic et de thérapie selon les revendications précédentes, caractérisé en ce que lesdites butées d'arrêt (28) sont pourvues de capteurs de pression pour mesurer la poussée du sujet.

7. Dispositif de diagnostic et de thérapie selon les revendications précédentes, caractérisé en ce que lesdites butées d'arrêt (28) sont portées par des pistons oléodynamiques (30') fixés sur des structures de support rigides entraînées hydrauliquement (40) qui peuvent être convenablement déplacées et mises en position.

8. Dispositif de diagnostic et de thérapie selon les revendications précédentes, caractérisé en ce qu'une unité centrale électronique de traitement est présente qui est conçue pour recevoir les données détectées par les cellules de charge (19A, 19B) de la bande qui se déplace (18) ainsi que les valeurs des tractions à plusieurs niveaux du corps durant la thérapie de manière à déterminer la totalité du traitement.

9. Dispositif de diagnostic et de thérapie selon les revendications précédentes, caractérisé en ce qu'un système détectant la position d'un point de référence lié dans l'espace au corps du patient est utilisé de plus pour surveiller la posture orthostatique du patient.
